# EUROPEAN PATENT APPLICATION

(11) **EP 0 849 247 A1**
(43) Date of publication of application: **24.06.1998**
(21) Application number: 97120745.1
(22) Date of filing: 26.11.1997
(51) Int. Cl.: C07C 43/13, C12P 41/00, C09K 19/02

(54) **Optically active alcohol and process for the production thereof**

(30) Priority: 17.12.1996 JP 336532/96
(71) Applicant: MITSUBISHI GAS CHEMICAL COMPANY, INC., Tokyo 100-0005 (JP)
(72) Inventor: Mine, Takakiyo, Tsukuba-shi, Ibaraki-ken (JP); Yui, Tomoyuki, Tsukuba-shi, Ibaraki-ken (JP)
(74) Representative: Kraus, Walter, Dr.

(57) **Abstract**

A novel R-configuration or S-configuration optically active alcohol of the formula (1),

CF₃C*H(OH)(CH₂)ₘO(CH₂)ₙCH(CH₃)₂ (1)

wherein C* is an asymmetric carbon atom, m is an integer of 2 to 7, and n is an integer of 0 to 3;
and a process for the production thereof. The novel optically active secondary alcohol having a trifluoromethyl group on an asymmetric carbon atom and a branched alkyloxy group at a terminal, provided by the present invention, is useful as a raw material for the production of a novel liquid crystal compound.

## Description

### Field of the Invention

The present invention relates to a novel optically active secondary alcohol having a trifluoromethyl group on an asymmetric carbon atom and a branched alkyloxy group at a terminal, and a process for the production thereof.

### Prior Art

Optically active compounds have been and are used in the fields of medicaments and agrochemicals, and in recent years, they are attracting attention as functional materials for ferrroelectric liquid crystals and organic non-linear materials.

For example, in the field of organic non-linear materials, an asymmetric center is desirably present in the molecule so that an organic material can produce a secondary non-linear optical effect (see, e.g., Yamaguchi, Nakano, Fueno, "KAGAKU (Chemistry)" 42(11), 757 (1987)). Further, in the field of ferrroelectric liquid crystal compound, the liquid crystal molecule is required to have an optically active structure in order to allow a liquid crystal compound to show ferroelectric characteristics (see, e.g., Jyono & Fukuda, "YUKI GOSEIKAGAKU KYOKAISHI (Journal of Organic Synthesis Chemistry Society", 47(6), 568 (1989)).

In recent years, further, anti-ferroelectric liquid crystal compounds are attracting remarkable attention, but the liquid crystal molecule of the anti-ferroelectric liquid crystal compound is also required to have an optically active structure, like the above ferrroelectric liquid crystal compound.

In the above fields, optically active 2-butanol, 2-octanol, 2-methyl-1-butanol and an amino acid derivative have been used as optically active sources. However, the characteristics of the obtained materials are limited so long as the above optically active materials are used.

In recent years, in the field of the ferroelectric liquid compounds, attempts have been vigorously made to synthesize ferroelectric liquid crystal compounds from the following alcohols which have a fluoroalkyl group substituted on an asymmetric carbon atom (C*), as an optically active source (see, e.g., JP-A-64-3154, JP-A-1-316339, JP-A-1-316367, JP-A-1-316372, JP-A-2-225434 and JP-A-2-229128).
(1) CF₃C*H(OH)CH₂COOC₂H₅
(2) CF₃C*H(OH)CH₂CH₂OC₂H₅
(3) CF₃C*H(OH)CH₂CH₂CH₂OC₂H₅
(4) CF₃C*H(OH)C₆H₁₃
(5) CF₃C*H(OH)C₈H₁₇
(6) C₂F₅C*H(OH)C₈H₁₇

All of anti-ferroelectric liquid crystal compounds derived from the above alcohols give high spontaneous polarization and give a relatively fast response speed since they have a fluoroalkyl group having a high electronegativity substituted on the asymmetric carbon atom.

It is also known that among the above alcohols, liquid crystal compounds derived from (4) CF₃C*H(OH)C₆H₁₃, (5) CF₃C*H(OH)C₈H₁₇ and (6) C₂F₅C*H(OH)C₈H₁₇, etc. easily give liquid crystals having an anti-ferroelectric liquid crystal phase, and hence, these are attracting attention as particularly characteristic alcohols.

Further, with regard to the method of synthesizing the optically active alcohol of CF₃C*H(OH)(CH₂)ₘOCₙH₂ₙ₊₁ in which m is an integer of 2 to 7 and n is an integer of 1 to 4, the present inventors conducted detailed studies on a new method of producing the intended optically active alcohol having a high optical purity without using ethyl trifluoroacetoacetate which is an expensive material, and a liquid crystal compound derived therefrom, and as a result, it was found that there could be obtained a greatly useful anti-ferroelectric liquid crystal compound or a ferrielectric liquid crystal compound both of which were not known before (see JP-A-5-65486 and JP-A-8-33555).

However, it has been found that an anti-ferroelectric liquid crystal compound or a ferrielectric liquid crystal compound derived from the above optically active alcohol has the following problems.
(i) Tilt angle is small.
(ii) The upper limit temperature of an anti-ferroelectric phase or a ferrielectric phase is low.
(iii) Viscosity is high.

There is therefore demanded a novel optically active alcohol which can provide a liquid crystal compound with characteristics which can overcome the above problems.

Presumably, an optically active secondary alcohol can be produced by various methods.

From the economic point of view, the use of an optically active compound as a starting material is not proper since the optically active compound is expensive.

An optically active secondary alcohol can be also produced by a method by asymmetric synthesis. For obtaining an optically active alcohol, for example, a method is conceivable which comprises preparing a corresponding ketone as a precursor and then, asymmetrically reducing the ketone in the presence of an asymmetrically reducing catalyst. In this case, however, the asymmetrically reducing catalyst is very expensive, a product having a high optical purity cannot necessarily be obtained, and only either one of an R-configuration and S-configuration optically active alcohol is obtained.

It is also conceivable to asymmetrically hydrolyze a proper ester as a precursor for an optically active compound, such as an acetate. An enzyme can be used as an effective asymmetric hydrolysis agent. The asymmetric hydrolysis of an acetate with lipase has been proposed by Kitazume et al (see T. Kitazume et al., J. Org. 52, 3211 (1987), JP-A-2-282304).

According to Kitazume et al, the acetate of the formula, CF₃CH(OCOCH₃)CₙH₂ₙ₊₁ in which n is an integer of 4 to 8 is asymmetrically hydrolyzed in the presence of lipase MY in a phosphate buffer solution.

However, the capability of lipase MY for recognizing asymmetry is greatly dependent upon differences in the chemical structure of a compound to be hydrolyzed. And, the optical purity of obtained hydrolysis products considerably varies from 55 to 98 ee% depending upon differences in the chemical structures as shown in Table 1 in the above literature by Kitazume et al.

The above results show that it is difficult to estimate whether or not an intended compound can be effectively asymmetrically hydrolyzed and that it is found only after a reaction whether or not an intended alcohol having a high optical purity can be obtained.

Further, there is another serious problem in that the capability for asymmetry recognition is not at all exhibited when some kinds of substituents are on an asymmetric carbon atom.

For example, lipase MY exhibits the remarkably high capability for asymmetry recognition in the asymmetric hydrolysis of CF₃C*H(OCOCH₃)(CH₂)₅OC₂H₅. However, lipase MY does not show any asymmetry recognition for an ester of a secondary alcohol CH₃CH(OCOCH₃)C₆H₁₃ having a methyl group substituted on the asymmetric carbon atom.

In addition to the above-mentioned methods, an optically active secondary alcohol is also produced by a method in which a secondary racemic alcohol is asymmetrically trans-esterified in the presence of a proper enzyme to carry out the optical resolution.

One example is a reaction of asymmetric trans-esterification of a secondary racemic alcohol in an organic solvent in the presence of a lipase (derived from porcine pancreas) (see A. M. Klibanov et al., J. Am. Chem. Soc. 1985, 106, 7072).

However, no lipase having high activity and high enantio-selectivity has been known. The asymmetric hydrolysis and the optical resolution by the asymmetric trans-esterification using an enzyme are advantageous in that both R-configuration and S-configuration optically active alcohols are easily obtained.

The present invention has been made in view of the above circumstances. It is an object of the present invention to provide a novel optically active secondary alcohol having a trifluoromethyl group on an asymmetric carbon atom and having a branched alkyloxy group at a terminal; and a process for the advantageous production thereof.

Studies by the present inventors have revealed that the above object of the present invention is achieved by an R-configuration or S-configuration optically active alcohol of the formula (1),

CF₃C*H(OH)(CH₂)ₘO(CH₂)ₙCH(CH₃)₂ (1)

wherein C* is an asymmetric carbon atom, m is an integer of 2 to 7, and n is an integer of 0 to 3.

The optically active alcohol of the above formula (1) in which m is 5 or n is 0 is preferred since it gives an anti-ferroelectric liquid crystal compound having excellent characteristics.

Further, according to the studies by the present inventors, a process for advantageously producing the optically active alcohol of the above formula (1) has been found. That is, according to the present invention, there is provided a process for the production of the optically active alcohol of the formula (1), which comprises the following steps (1) to (4),
step (1): which comprises converting a halogen compound of the formula (2) to a Grignard reagent,

   X(CH₂)ₘO(CH₂)ₙCH(CH₃)₂ (2)

   wherein m and n are as defined in the formula (1) (m and n in formulae to be described later also have the same meanings as those in the formula (1)), and X is a halogen atom other than a fluorine atom,
   and then reacting the Grignard reagent with a trifluoroacetic acid metal salt of the formula (3) or (4),

   CF₃COOM¹ (3)

   (CF₃COO)₂M² (4)

   wherein M¹ is Li, Na or K, and M² is Mg or Ca, to form a ketone of the formula (5),

   CF₃CO(CH₂)ₘO(CH₂)ₙCH(CH₃) (5)
step (2): which comprises reducing the ketone of the formula (5) to form a racemic alcohol of the formula (6),

   CF₃CH(OH)(CH₂)ₘO(CH₂)ₙCH(CH₃)₂ (6)
step (3): which comprises reacting the alcohol of the formula (6) with a halide or anhydride of an aliphatic carboxylic acid whose alkyl portion has 1 to 5 carbon atoms, to form an ester of the formula (7),

   CF₃CH(OCOR)(CH₂)ₘO(CH₂)ₙCH(CH₃)₂ (7)

   wherein R is an alkyl group having 1 to 5 carbon atoms, and
step (4): which comprises carrying out an asymmetric hydrolysis of the ester of the formula (7) with lipase, then, after the completion of the asymmetric hydrolysis, bringing the reaction mixture into contact with an organic solvent compatible with water to precipitate the lipase, recovering the lipase by filtration, and separating an optically active alcohol and an optically active ester from the filtrate.

In the process of the present invention, the trifluoroacetic acid metal salt of the formula (3) or (4) to be reacted with the Grignard reagent in the step (1) is preferably used in the form of a solution thereof in tetrahydrofuran. Further, the reaction for obtaining the ketone in the step (1) is preferably carried out at a temperature between 20°C and 50°C.

In the step (2), it is preferred to use an NaBH₄ aqueous solution containing sodium hydroxide as a reducing agent for the reduction of the ketone. The reduction of the ketone is preferably carried out at a temperature between 20°C and 40°C.

In the step (3), the alkyl portion of the aliphatic carboxylic acid has 1 to 5 carbon atoms, preferably 1 to 3 carbon atoms. And, the halide or anhydride of the aliphatic carboxylic acid includes acetic acid chloride, propionic acid chloride, butyric acid chloride, acetic anhydride, propionic anhydride, and the like. The step (3) is preferably carried out at a temperature between 60°C and 90°C.

As a lipase used in the step (4), lipase derived from porcine pancreas is preferred, and lipase MY is particularly advantageously used. The lipase MY is commercially available, for example, from MEITO SANGYO CO., LTD. and is easily obtained.

In the step (4), preferably, the asymmetric hydrolysis with lipase is carried at a temperature between 20°C and 40°C, and the molar amount of water used is 20 to 50 times the molar amount of the ester.

Further, in the step (4), the reaction mixture after the asymmetric hydrolysis with the lipase is brought into contact with an organic solvent of which the amount is 5 to 20 times the amount of the water used for the asymmetric hydrolysis, to precipitate the lipase. The precipitated lipase is recovered by filtration. The recovered lipase is dried under reduced pressure and can be recycled in the step (4), and the optically active alcohol can be therefore more economically produced. The above organic solvent is preferably at least one member selected from the group consisting of acetone, methanol, ethanol and tetrahydrofuran. Further, the optically active ester separated in the step (4) can be converted to an optically active alcohol by a known hydrolysis method.

### Examples

The present invention will be explained in detail with reference to Examples hereinafter, while the present invention shall not be limited thereto.

### Example 1 (Preparation of R-(+)-1,1,1-trifluoro-2-hydroxy-7-(1-methylethoxy)heptane (E1), formula (1) in which m = 5, n = 0)

### (1) Preparation of 1-bromo-5-(1-methylethoxy)pentane

13 Grams of metal sodium was little by little added under stirring to 300 g of isopropyl alcohol under a nitrogen gas current. Then, the mixture was refluxed under heating for one hour to allow the sodium to react completely. While the reaction mixture was heated with taking care not to precipitate a solid, a solution of 50 % of 1,5-dibromopentane in isopropyl alcohol was dropwise added. Then, the mixture was stirred at room temperature for 15 hours. After the completion of the reaction, water was added, and the mixture was extracted with ether. An ether layer was washed with a hydrochloric acid aqueous solution and with a saturated sodium chloride aqueous solution, and dried over anhydrous sodium sulfate.

The ether layer was distilled off, and then the residue was distilled (83 to 87°C, 10 Torr) to give 50 g of a mixture (purity 50 %) of an end product with 1,5-dibromopentane.

### (2) Synthesis of 1,1,1-trifluoro-2-hydroxy-7-(1-methylethoxy)heptane

6.5 Grams (270 mmol) of metal magnisum was placed in a round-bottom flask, atmosphere in the flask was replaced by a nitorgen gas, and then 100 ml (milliliter) of dry tetrahydrofuran was added. To the resultant mixture was dropwise added a solution of 46 g (110 mmol) of 1-bromo-5-(1-methylethoxy)pentane in 100 ml of dry tetrahydrofuran so as to lower the reaction temperature to 40°C or lower. The reaction mixture was aged for 1 hour, and then 200 g (280 mmol) of magnesium bis(trifluoroacetate) (35 % tetrahydrofuran solution) was dropwise added so as to keep the reaction temperature to 50°C or lower. Then, the mixture was allowed to react at 50°C for 2 hours. After the completion of the reaction, 150 ml of 6N hydrochloric acid was added, then the mixture was stirred at room temperature for 2 hours, and an organic layer was separated.

28 Grams of 12 % NaBH₄ (NaOH aqueous solution) was gradually added to the organic layer. The mixture was stirred at room temperature for 3 hours, then water was added, and the mxiture was extracted with ether. An ether layer was washed with 6N hydrochloric acid and then washed with water until it became neutral, and the ether layer was further washed with a saturated sodium chloride aqueous solution.

The ether layer was dried over anhydrous sodium sulfate, and the ether was distilled off to give a crude product. The crude product was purified by distillation (90 to 110°C, 6 Torr) to give 20 g of an end product (purity by analysis using gas chromatography (GC purity) 50 %).

### (3) Synthesis of 1,1,1-trifluoro-2-acetoxy-7-(1-methylethoxy)heptane

10 Grams (98 mmol) of acetic anhydride and 10 g (130 mmol) of pyridine were added to 17 g (38 mmol) of the 1,1,1-trifluoro-2-hydroxy-7-(1-methylethoxy)heptane obtained in the above (2), and then, the mixture was stirred at room temperature for 30 hours. 10 ml of water was added, and the mixture was stirred for 10 hours. Further, water was added, and the mixture was extracted with ether. An ether layer was washed with 6N hydrochloric acid, then washed with water until it became almost neutral, and further, washed with a saturated sodium chloride aqueous solution. The ether layer was dried over anhydrous sodium sulfate, and the ether was distilled off to give 18 g of a crude product. The crude product was purified by distillation (70°C, 3 Torr) to give 11 g of an end product (GC purity 90 %).

### (4) Synthesis of R-(+)-1,1,1-trifluoro-2-hydroxy-7-(1-methylethoxy)heptane

30 Grams of water and 8.2 g of lipase MY (supplied by MEITO SANGYO CO., LTD.) were added to 6.7 g (22 mmol) of the acetyl compound obtained in the above (3), and the mixture was stirred at room temperature for 8 hours. Then, 150 ml of acetone was added, and the mixture was stirred for 1 hour. Precipitated lipase was separated by filtration, and then the acetone was distilled off.

To the resulting mixture was added water, and the mixture was extracted with ether. An ether layer was washed with water several times and, further, washed with a saturated sodium chloride aqueous solution. The ether layer was dried over anhydrous sodium sulfate, and then the ether was distilled off, to give 7 g of a crude product.

The above crude product was purified by silica gel column chromatography to separate it into R-(+)-1,1,1-trifluoro-2-hydroxy-7-(1-methylethoxy)heptane and S-(-)-1,1,1-trifluoro-2-acetoxy-7-(1-methylethoxy)heptane. The yield of the R-(+) configuration compound was 1.8 g.

On the other hand, the S-(-)-1,1,1-trifluoro-2-acetoxy-7-(1-methylethoxy)heptane was hydrolyzed to give S-(-)-1,1,1-trifluoro-2-hydroxy-7-(1-methylethoxy)heptane.

Table 1 shows NMR data of the R-(+) configuration compound, and Table 2 shows the specific rotatory power and optical purity thereof.

The optical purity and the specific rotatory power were determined as follows.

The R-(+) configuration optically active alcohol obtained in the above (4) was converted to an acetate with pyridine/acetic anhydride. The resultant acetate was analyzed with a gas chromatograph (CP Cyclodex β236M) used for the analysis of optically active materials, and the purity was determined on the basis of a peak area ratio of two enathiomers. Further, chloroform was used as a solvent and an optical rotation meter was used to determine the specific rotatory power.

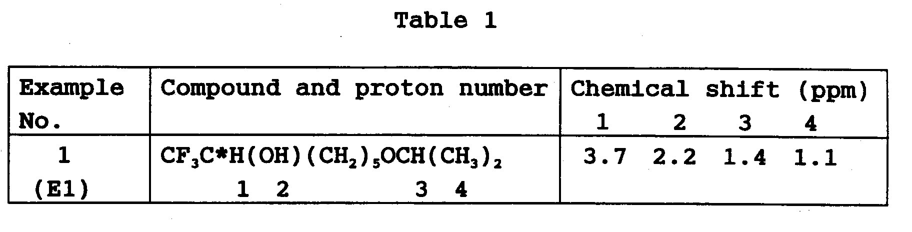

**Table 2**

| Example | Chemcial structure | Optical purity | Specific rotatory power*1 |
|---|---|---|---|
| 1 (E1) | CF₃C*H(OH)(CH₂)₅OCH(CH₃)₂ | >99 %ee | +20.2° |

| | | | |
|---|---|---|---|
| *1: Measured with sodium D ray at 29°C. | | | |

The present invention provides novel optically active secondary alcohols having a trifluoromethyl group on an asymmetric carbon atom and a branched alkyloxy group at a terminal, and a process for producing them economically and easily.

## Claims

1. An R-configuration or S-configuration optically active alcohol of the formula (1),
CF₃C*H(OH)(CH₂)ₘO(CH₂)ₙCH(CH₃)₂ (1)
wherein C* is an asymmetric carbon atom, m is an integer of 2 to 7, and n is an integer of 0 to 3.

2. The optically active alcohol of claim 1, which has the formula (1) in which m is 5.

3. The optically active alcohol of claim 1 or 2, which has the formula (1) in which n is 0.

4. A process for the production of the optically active alcohol of the formula (1), which comprises the following steps (1) to (4),
step (1): which comprises converting a halogen compound of the formula (2) to a Grignard reagent,
X(CH₂)ₘO(CH₂)ₙCH(CH₃)₂ (2)
wherein m and n are as defined in the formula (1) (m and n in formulae to be described later also have the same meanings as those in the formula (1)), and X is a halogen atom other than a fluorine atom,
and then reacting the Grignard reagent with a trifluoroacetic acid metal salt of the formula (3) or (4),
CF₃COOM¹ (3)
(CF₃COO)₂M² (4)
wherein M¹ is Li, Na or K, and M² is Mg or Ca, to form a ketone of the formula (5),
CF₃CO(CH₂)ₘO(CH₂)ₙCH(CH₃) (5)
step (2): which comprises reducing the ketone of the formula (5) to form a racemic alcohol of the formula (6),
CF₃CH(OH)(CH₂)ₘO(CH₂)ₙCH(CH₃)₂ (6)
step (3): which comprises reacting the alcohol of the formula (6) with a halide or anhydride of an aliphatic carboxylic acid whose alkyl portion has 1 to 5 carbon atoms, to form an ester of the formula (7),
CF₃CH(OCOR)(CH₂)ₘO(CH₂)ₙCH(CH₃)₂ (7)
wherein R is an alkyl group having 1 to 5 carbon atoms, and
step (4): which comprises carrying out an asymmetric hydrolysis of the ester of the formula (7) with lipase, then, after the completion of the asymmetric hydrolysis, bringing the reaction mixture into contact with an organic solvent compatible with water to precipitate the lipase, recovering the lipase by filtration, and separating an optically active alcohol and an optically active ester from the filtrate.

5. The process of claim 4, wherein the trifluoroacetic acid metal salt in the form of a tetrahydrofuran solution is reacted with the Grignard reagent in the step (1).

6. The process of claim 4, wherein the step (2) uses an NaBH₄ aqueous solution containing sodium hydroxide is used as a reducing agent for the reduction of the ketone.

7. The process of claim 4, wherein the asymmetric hydrolysis with the lipase in the step (4) is carried out at a temperature between 20°C and 40°C.

8. The process of claim 4, wherein the asymmetric hydrolysis with the lipase in the step (4) uses water in a molar amount 20 to 50 times the molar amount of the ester.

9. The process of claim 4, wherein a reaction mixture from the asymmetric hydrolysis with the lipase is brought into contact with an organic solvent in an amount 5 to 20 times the amount of water used for the asymmetric hydrolysis, to precipiate the lipase, and the precipitated lipase is recovered by filtraion, dried under reduced pressure and recylced to the step (4).

10. The process of claim 4, wherein the organic solvent is at least one member selected from the group consisting of acetone, methanol, ethanol and tetrahydrofuran.
